# EUROPEAN PATENT APPLICATION

(11) **EP 2 062 966 A1**
(43) Date of publication of application: **27.05.2009**
(21) Application number: 07807037.2
(22) Date of filing: 11.09.2007
(51) Int. Cl.: C12N 1/14, A01N 63/00, A01P 3/00, C12N 15/09

(54) **MATERIAL FOR CONTROL OF SOIL-BORNE DISEASE IN PLANT UTILIZING NOVEL FILAMENTOUS FUNGI**

(30) Priority: 15.09.2006 JP 2006251418
(71) Applicant: Idemitsu Kosan Co., Ltd., Chiyoda-ku Tokyo 100-8321 (JP); National University Corporation Tokyo University of Agriculture and Technology, Fuchu-shi, Tokyo 183-8538 (JP)
(72) Inventor: KAWABATA, Takahiro, Sodegaura-shi Chiba 299-0293 (JP); ABE, Hiroshi, Fuchu-shi Tokyo 183-8538 (JP); NARITA, Yasusaburo, Iwamizawa-shi Hokkaido 068-0027 (JP)
(74) Representative: Vossius & Partner
(86) International application number: PCT/JP2007/067628
(87) International publication number: WO 2008/032693

(57) **Abstract**

Disclosed is a novel filamentous fungus SD-F06 strain which can infect a root of a plant and grow symbiotically with the plant and is capable of controlling a soil-borne disease. Also disclosed is a material for controlling a soil-borne disease, which comprises a fungus body or fungal culture of a filamentous fungus SD-F06 strain. Further disclosed is a method for controlling a soil-borne disease in a plant by utilizing the material.

## Description

### Technical Field

The present invention relates to a novel strain (SD-F06 strain) which has an effect of controlling a soil-borne disease, in particular a disease caused by a fungus belonging to the genus *Gaeumannomyces* or *Fusarium*, by mixed application of the novel strain to a field of a plant of interest and/or a soil of a seedbed, and a material for controlling a soil-borne disease comprising a fungus body or a fungal culture of the novel strain, as an active ingredient.

### Background Art

Soil-borne diseases are extremely difficult to be controlled once the diseases develop, and soil-borne diseases are serious diseases that can cause a collapse of growing district. Among those, a soil-borne disease by a fungus belonging to the genus *Fusarium* causes a heavy damage to vegetables, which includes tomato wilt disease, spinach wilt disease, lettuce root rot disease, cabbage chlorosis, strawberry chlorosis, or Fusarium wilt of cucumber, melon, watermelon, or the like, and is regarded as one of the most serious diseases. At present, soil fumigation with chemical pesticide such as methyl bromide and chloropicrin is regarded as the most effective technology. However, methyl bromide is regarded as a global warming factor and is restricted in its production and use. Further, there have been pointed out a concern related to health of neighboring residents due to the usage of a gas agent and a problem of causing a soil cavitation of killing useful microorganisms or natural enemy insects in the soil, thereby increasing a damage caused by a pathogen which has newly invaded (resurgence phenomenon). Therefore, there is desired an invention of a microbial pesticide which is safe and environmentally-friendly, and has a stable effect.

A Sterile Dark fungus (K89 strain) described in Non-patent Document 1 and 2 is a wheat root symbiotic fungus, and a controlling effect thereof against wheat damping-off disease (*Gaeumannomyces* fungus disease) has been recognized. When *Gaeumannomyces graminis* and the Sterile Dark strain have been placed in a pot at the same time, the pathogenesis of the damping-off disease has been ameliorated, but the effect has not been sufficient. In addition, in the case of an actual field, a practical realization of the Sterile Dark fungus as the microbial pesticide has been difficult in terms of cost, because extremely large amount of materials were needed under the application condition of 1.5 to 2.5% (V/V) soil incorporation as described in Non-patent Document 1. Further, there is no study on a controlling effect against a disease caused by a fungus belonging to the genus *Fusarium*, which is the most serious soil-borne disease of vegetables.
Further, in addition to the Sterile Dark fungus, there have been attempted a number of methods of biological control of a soil-borne disease by inoculating a soil or a plant with microorganisms (bacteria and filamentous fungi) which have controlling action against a pathogen, such as nonpathogenic fungus *Fusarium* (Non-patent Document 3), *Bacillus* bacterium (Patent Documents 1 and 2), *Pseudomonas* bacterium (Patent Documents 5 and 6), and fungus *Trichoderma* (Patent Documents 3 and 4). However, there are problems that it is difficult to allow the applied bacteria or fungi to live stably in the soil and the controlling effect against a soil-borne disease is unstable, and that, even in the case of endophytic fungi within plants such as nonpathogenic fungus *Fusarium*, the controlling effect persists only for a short period of time. Thus, a sufficient controlling effect against a disease has not yet been achieved.

Patent Document 1: JP 03-128988 A *(Bacillus* bacterium)
Patent Document 2: JP 04-117278 A (*Bacillus* bacterium)
Patent Document 3: JP 01-102010 A (fungus *Trichoderma*)
Patent Document 4: JP 02-245178 A (fungus *Trichoderma*)
Patent Document 5: JP 02-35075 A (*Pseudomonas* bacterium)
Patent Document 6: JP 05-916 A *(Pseudomonas* bacterium)
Non-patent Document 1: "Effective utilization technology of useful microorganisms," Soil and Rhizosphere IV, p. 131-136, 1986
Non-patent Document 2: Ann. Phytopath. Soc. Japan 57: 301-305, 1991 Non-patent Document 3: "A control of Fusarium wilt of sweet potato caused by nonpathogenic Fusarium oxysporum and practical realization thereof, " The Japan Agricultural Technology Monthly, October issue, p. 19-22 (2003)

### Disclosure of the Invention

An object of the present invention is to provide a material, which is not affected by a microbial group in a soil and exhibits a stable controlling effect against a soil-borne disease with a small amount of application, and to provide a method of controlling a soil-borne disease in a plant using the material.

The present invention provides a novel filamentous fungus SD-F06 strain, which is capable of growing symbiotically with a root of a plant and has an ability of controlling a soil-borne disease. The present invention also provides a material for controlling a soil-borne disease which comprises a fungus body or fungal culture of the filamentous fungus SD-F06 strain, and a method of controlling a soil-borne disease in a plant by using the material.

That is, the summary of the present invention are as the followings.
(1) A filamentous fungus SD-F06 strain (FERM BP-10841) or a mutant strain thereof, which has an ability of infecting a root of a plant and growing symbiotically with the plant and has an effect of controlling a soil-borne disease developed in the plant.
(2) A material for controlling a soil-borne disease, comprising a fungus body or a fungal culture of the SD-F06 strain or the mutant strain thereof according to (1), as an active ingredient.
(3) A method of controlling a soil-borne disease, comprising treating a soil for growing a plant with the material for controlling a soil-borne disease according to (2).
(4) Thematerial for controlling a soil-borne disease according to (2), wherein the soil-borne disease is caused by a phytopathogenic filamentous fungus.
(5) The method of controlling a soil-borne disease according to (3), wherein the soil-borne disease is caused by a phytopathogenic filamentous fungus.
(6) The material for controlling a soil-borne disease according to (4), wherein the disease caused by a phytopathogenic filamentous fungus is a disease caused by one or more kinds of fungi which belong to genera *Fusarium, Gaeumannomyces, Rhizoctonia, Pythium, Verticillium, Phytophthora, Sclerotium, Corticium, Plasmodiophora, Rhizopus, Trichoderma, Microdochium*, and *Sclerotinia*.
(7) The method of controlling a soil-borne disease according to (5), wherein the disease caused by a phytopathogenic filamentous fungus is a disease caused by one or more kinds of fungi which belong to genera *Fusarium, Gaeumannomyces, Rhizoctonia, Pythium, Verticillium, Phytophthora, Sclerotium, Corticium, Plasmodiophora, Rhizopus, Trichoderma, Microdochium*, and *Sclerotinia*.

### Effects of the Invention

(1) The novel plant symbiotic fungus SD-F06 strain of the present invention obtained from field soil of Hokkaido can grow symbiotically with various plants at the inside of the root, and has an ability to control a soil-borne disease from which the symbiotic plant suffers.
(2) The SD-F06 strain of the present invention can grow symbiotically with a wide range of plant species, and can be applied to the control of soil-borne diseases in an extremely wide range of plant species.
(3) The SD-F06 strain of the present invention shows high controlling effect against the disease caused by the genus *Fusarium*, which is the most serious soil-borne disease of vegetables.
(4) The material containing SD-F06 strain of the present invention is mixed into culture soil for raising seedling and thus symbiotic seedlings are produced, whereby the material exhibits a stable controlling effect without being influenced by groups of microorganisms in the soil after the symbiotic seedlings are transplanted to the field. Further, the symbiosis of the fungus with the plant is maintained for one month or more.
(5) As an amount of SD-F06 fungus material of the present invention to be mixed into the culture soil for raising seedling, 1% (W/V) or less is sufficient. An excellent controlling effect can be obtained with an extremely small application amount of the material, and thus a controlling material having high economical efficiency can be provided.

### Brief Description of the Drawing

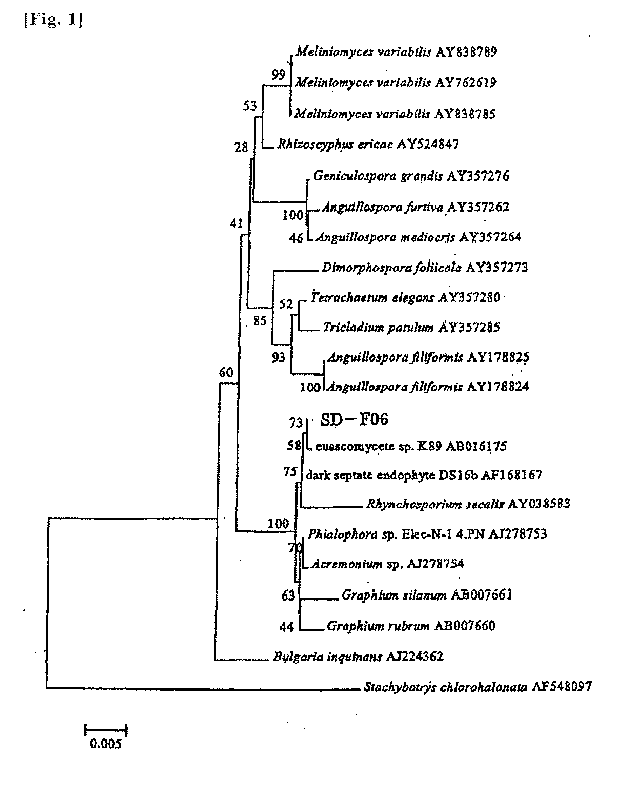

Fig. 1 shows a molecular phylogenetic tree of the SD-F06 strain, which is produced by a neighbor-joining method. A line at bottom left of the tree represents a scale bar.

### Description of the Preferred Embodiments

Hereinafter, the present invention is described in detail.
A novel filamentous fungus SD-F06 strain of the present invention can grow symbiotically with various plants at the inside of the root, and has an ability to control a soil-borne disease.
The novel filamentous fungus SD-F06 strain of the present invention was obtained by screening symbiotic fungi at the inside of the plant root from the soil of continuous cropping field of wheat in Hokkaido. Features of the SD-F06 strain were as follows: colonies on each of a potato dextrose agar medium, a malt extract agar medium, and an oatmeal agar medium were brown and velvet-like; and no spore formation was observed on the agar media. Thus, morphological classification of the novel filamentous fungus SD-F06 strain was difficult.
The filamentous fungus SD-F06 strain of the present invention was subjected to 18S rRNA analysis and it was revealed that the sequence of the filamentous fungus SD-F06 strain has a homology of 99.8% to the sequence of dark septate endophyte DS16b, Sterile DarkK89 fungus (euascomycete sp. K89) by 18S rRNA analysis. Although the filamentous fungus SD-F06 strain was a new strain belonging to the *Ascomycetes*, the taxonomic position after the *Ascomycetes* is uncertain. An evolutionary tree of the SD-F06 strain is shown in Fig. 1.
The novel filamentous fungus SD-F06 strain of the present invention is deposited as FERM BP-10841 on July 26, 2006, at National Institute of Advanced Industrial Science and Technology, International Patent Organism Depositary (Central 6, 1-1-1 Higashi Tsukuba, Ibaraki, 305-8566, Japan).

The strain or a mutant strain of the present invention is a SD-F06 strain or a mutant strain thereof which can infect various plants at the inside of the root and grow symbiotically with the plants, and has an ability to control a soil-borne disease.
The "mutant strain" of the present invention includes any mutant strain derived from the SD-F06 strain, as long as the mutant strain is a strain which, as mentioned above, has an ability of infecting a root of a plant and growing symbiotically with the plant and has an effect of controlling a soil-borne disease developed in the plant. The mutant strain can be obtained by: mutagenizing the SD-F06 strain with artificial mutation means using ultraviolet irradiation, X-ray irradiation, a mutagenetic agent (e.g., N-methyl-N-nitro-N-nitrosoguanidine), or the like; and selecting a strain having a controlling effect against the disease. Further, a natural mutant strain of the SD-F06 strain is also included in the mutant strain, as long as the natural mutant strain has a controlling effect against a disease.
It should be noted that the term "SD-F06 strain" in the description below is sometimes used in the meaning of "SD-F06 strain or the mutant strain thereof".

In the present invention, the phrase "having an ability of infecting a root of a plant and growing symbiotically with the plant" means that the SD-F06 strain grows or proliferates in the root of the growing plant under appropriate conditions and that the SD-F06 strain does not provide the plant with adverse effects, such as deformation or discoloration of the tissue of the plant.
Whether or not a strain has an ability of infecting a root of a plant and growing symbiotically with the plant can be judged by: applying fungus bodies of the strain to the plant body or a soil in which the plant is grown; growing the plant for a month under appropriate conditions; and confirming whether or not the strain grows or proliferates in the root of the plant and is free from providing the plant with adverse effects, such as deformation or discoloration of the tissue of the plant. Whether or not a strain grows or proliferates in a root of a plant can be confirmed by using a method described in Example 2 to be mentioned later. Further, whether or not the strain is free from providing the plant with adverse effects, such as deformation or discoloration of the tissue of the plant, can be confirmed by visual observation and the like.
The term "plant" as used in the phrase "having an ability of infecting a root of a plant and growing symbiotically with the plant" is not particularly limited as long as the "plant" is a plant which the novel filamentous fungus SD-F06 strain of the present invention can infect and grow symbiotically with. The SD-F06 strain of the present invention can grow symbiotically with a wide range of plant species, and even when listing the already-confirmed plant species, there are many species such as Poaceae plants (wheat, oat, corn, lawn grass, and pasture grass), Cucurbitaceae plants (watermelon and melon), Solanaceae plants (tomato, eggplant, and potato), Leguminosae plants (soybean, azuki, and haricot), Brassicaceae plants (chineise cabbage), Asteraceae plants (lettuce), Chenopodiaceae plants (sugar beet), Liliaceae plants (asparagus and leek), and Iridaceae plants (freesia). Therefore, the filamentous fungus SD-F06 strain can be applied to control a soil-borne disease in an extremely wide range of plants, such as Polygonaceae plants, Convolvulaceae plants, Malvaceae plants, Araceae plants, Umbelliferae plants, Zingiberaceae plants, Lamiaceae plants, Rosaceae plants, Dioscoreaceae plants, Campanulaceae plants, Scrophulariaceae plants, Primulaceae plants, Caryophyllaceae plants, Orchidaceae plants, and Gentianaceae plants, as well as the plants belonging to the above-mentioned respective families.

In the present invention, the strain "having an effect of controlling a soil-borne disease developed in a plant, with which the strain infects and grows symbiotically" means a strain which has an effect of preventing or curing the disease of the plant.
The phrase "strain which has an effect of preventing a disease of a plant" used herein refers to a strain as follows: when plants, which can be infected with a pathogen, are grown for a month in a soil containing the pathogen that causes the disease of the plants under the same appropriate conditions except that the strain is applied to the plants, the disease incidence of the plants to which the strain is applied is lower than that of the plants to which the strain is not applied (see Example 4 to be described later). Further, the "strain which has an effect of curing a disease of a plant" used herein refers to a strain causing the following case: when plants, which has been infected with the disease of the plants, are grown for a month under the same appropriate conditions except that the strain is applied to the plants, the disease degree of the plants to which the strain is applied becomes less than that of the plants to which the strain is not applied.
The phrase "strain having an effect of controlling a disease in a plant" of the present invention, specifically, means that, for example: a preventive value is generally 50% or more, preferably 60% or more, and more preferably 90% or more when an experiment shown in Example 4, which is to be mentioned later, is performed.

The term "disease in a plant" of the present invention is not particularly limited as long as it is a plant disease against which the novel filamentous fungus SD-F06 strain of the present invention exhibits its controlling effect. A disease in plant which is caused by being infected with a pathogen is preferred, and a soil disease is more preferred. In the present invention, the soil disease to be controlled is preferably a soil-borne disease, and more specifically, a disease caused by one or more kinds of fungi which belong to genera *Fusarium, Gaeumannomyces, Rhizoctonia, Pythium, Verticillium, Phytophthora, Sclerotium, Corticium, Plasmodiophora, Rhizopus, Trichoderma, Microdochium*, and *Sclerotinia*, but the disease is not limited thereto.

The material for controlling a soil-borne disease of the present invention is a material containing a fungus body or fungal culture of the SD-F06 strain, which allows the SD-F06 strain to infect a root of a plant and grow symbiotically with the plant and is capable of controlling a soil-borne disease by mixing the material into the soil at the time of growing various plants.
The SD-F06 strain of the present invention can be cultured in the same manner as a general method of culturing a microorganism. For example, in the case of a laboratory experiment, a culture method of culturing the SD-F06 strain on a potato dextrose agar medium for 10 days at 23°C is given. In the case of a large-scale culture, a general liquid culture or a solid culture, which uses a plant-derived solid component such as bran or oatmeal, or a porous body supplemented with a sugar or a nitrogen source, can be performed. The obtained culture can be used as the material of the present invention as it is or after being dried if required. In a preferred embodiment of the present invention, the material for controlling a soil-borne disease of the present invention can be produced by a method as described in Example 1.

The material of the present invention can be produced by simply suspending the fungus bodies of the SD-F06 strain of the present invention in a liquid such as water, or can also be produced as a formulation such as a liquid formulation, a powder formulation, a granular formulation, or an aerosol by blending another component. Examples of another component include a liquid carrier, a solid carrier, a surfactant (emulsifier, dispersant, antifoamer, or the like), and an adjuvant, and one, two, or more kinds of them can be used as a mixture.

Examples of the liquid carrier include a phosphate buffer, a carbonate buffer, and a physiological saline. Examples of the solid carrier include: natural mineral powders such as kaolin, clay, talc, chalk, quartz, attapulgite, montmorillonite, and diatom earths; synthetic mineral powders such as silicic acid, alumina, and silicate; and macro-molecule natural products such as crystalline cellulose, corn starch, gelatin, and alginic acid. Further, examples of the surfactants include polyoxyethylene-fatty acid ester, polyoxyethylene-fatty acid alcohol ether, alkylaryl polyglycol ether, alkylsulfonate, alkylsulfate, andarylsulfonate. Examples of the adjuvants include carboxymethylcellulose, polyoxyacethyleneglycol, gum arabic, starch, and lactose.

Further, in the case where the liquid formulation using an aqueous solvent as a carrier is produced, a water-soluble polymer may be added to improve hydration ability of the fungus bodies in the solvent. Examples of the water-soluble polymer include polyvinyl alcohol, polyethylene glycol, polyvinylmethylether, polyvinylamine, polyvinylpyrrolidone, polyethyleneimine, and polyacrylamide. In addition, polysaccharides such as xyloglucan and guar gum may be added to improve the adherability of the SD-F06 strain to a root of a plant and to enhance the stability of the SD-F06 strain in a formulation.

A concentration of the SD-F06 strain contained in the material of the present invention is not particularly limited as long as an effect of the present invention is not adversely affected, and the concentration in a formulation is 10² to 10⁸ CFU/g (Colony Forming Unit) and preferably 10⁴ to 10⁷ CFU/g (Colony Forming Unit). Further, the concentration can be appropriately changed depending on the controlling effect of the SD-F06 strain to be used.
Further, when the material of the present invention is used in a form of a granular formulation or a powder formulation, the material can be used by being diluted with water so that the weight of the resultant becomes 10 to 50,000 times and more preferably 100 to 10,000 times the weight of the material.
Further, the material of the present invention may contain, in addition to the above substances, any appropriate substances such as the medium used for culturing the SD-F06 strain which is contained in the material and the like, as long as the effect of the present invention is not impaired.

A method of using the material of the present invention is not particularly limited, and is appropriately selected depending on the forms of the formulation and the like, crops, and diseases, and examples of the method include a ground application of a liquid formulation, a solid ground application, an aerial application of a liquid formulation, a aerial application of a solid formulation, a submerged application, an intrafacility application, a soil mixing application, a soil irrigation application, surface treatment (seed coating, coating treatment, and the like), an application method using a seedling box, and methods such as uniflorous treatment and stock part treatment. Preferred examples include: coating seeds/seed tubers of a plant to be cultivated with materials of various formulations; subjecting flowers of the plant to be cultivated to uniflorous treatment; treating stems and leaves of the plant to be cultivated; subjecting wounded sites and pruned parts of the plant to be cultivated to coating treatment; performing soil irrigation; and performing soil mixing. Here, when the material of the present invention is applied to the soil, the plant to be cultivated may be planted after the material of the present invention is applied to the soil, or the material of the present invention may be applied to the soil after the plant to be cultivated is planted.

Further, when the material of the present invention is applied to the plant to be cultivated, there can be performed: a mixed application with an insecticide, a nematicide, an acaricide, a herbicide, a microbicide, a plant growth regulator, a fertilizer, a soil conditioner (e.g., peat, humic acid material, or polyvinylalcohol-based material), and the like; a simultaneous application with them without mixing; or a alternate application thereof.
An application amount of the material of the present invention is not particularly limited, and can be appropriately adjusted depending on kind of the disease, kind of the plant to be applied, a form of the material, and the like. For example, when ground application of the material of liquid formulation is performed, a concentration of the SD-F06 strain in the application liquid is generally 10² to 10⁶ CFU/mL (Colony Forming Unit) and preferably 10⁴ to 10⁷ CFU/mL (Colony Forming Unit), and the application amount of the material may be 0.5 to 100 L/a. Further, the granular formulation and the powder formulation may be subjected to application as they are without being diluted. When ground applications of the granular formulation, the powder formulation, and the like are performed, it is preferred that the application amount of the SD-F06 strain be about 10² to 10⁹ CFU/a.

### Examples

Hereinafter, the present invention is described in more detail by way of Examples, but the present invention is not limited to these Examples.

### Example 1

### Production of a material containing SD-F06 strain

50 ml of water was added to 100 g of commercially available oatmeal, and the mixture was sterilized at 121°C for 30 minutes, and thus a solid culture medium was produced. To the medium, a part of fungus bodies obtained by proliferating SD-F06 strain (FERM BP-10841) as an inoculum on an agar plate medium (potato dextrose agar medium) at 23°C for 2 weeks was added, and cultured at 23°C for 10 days, followed by air-drying at room temperature, whereby a solid culture of the SD-F06 strain was obtained. Then, the air-dried culture was pulverized by a mill, and the resultant was used as a SD-F06 strain material for the studies of Examples below.

### Example 2

### Confirmations of infection of SD-F06 strain to various plants and symbiosis of SD-F06 strain with various plants

A mixture in which the SD-F06 strain material produced in Example 1 was mixed at 0.5% (W/V) into commercially available gardening soil (Yosaku N-15: National Federation of Agricultural Cooperative Associations) was prepared, and the mixture was filled into a 32-well cell tray (Naesaku-kun, manufactured by KOBAYASHI & CO., LTD.) and was irrigated. After that, seeds of respective plants shown in Table 1 were sown therein and cultivated in a greenhouse at 18°C to 24°C. 25 days after sowing, seedlings of the respective plants were removed, a root of each of the seedlings was washed cleanly with running tap water for 5 minutes to remove the soil, and a surface of the root was sterilized with sodium hypochlorite (0.5% liquid). Then, the root was left standing still on a potato dextrose agar medium containing 50 ppm of streptomycin and after 2 weeks at 15°C, a formed colony was observed. A colony of the SD-F06 strain with blackish brown color is formed from a root of the seedling with which the SD-F06 strain grows symbiotically, thus, the seedling can be distinguished from a seedling with which the SD-F06 strain does not grow symbiotically. As a result, it was revealed that, as shown in Table 1, the SD-F06 strain was able to infect and grow symbiotically with the roots of remarkably many kinds of plants.

[Table 1]

**Table 1 Examples of plants with which SD-F06 strain grows symbiotically**

| Category | Name of plant |
|---|---|
| Cucurbitaceae | Watermelon, Melon, Bottle gourd |
| Solanaceae | Tomato, Eggplant, Potato |
| Brassicaceae | Chinese cabbage |
| Leguminosae | Soybean, Azuki, Haricot |
| Chenopodiaceae | Spinach, Sugar Beet |
| Poaceae | Wheat, Corn, Poaceae pasture grass |
| Liliaceae | Leek, Asparagus |
| Asteraceae | Lettuce |
| Other | Freesia |

### Example 3

### Test of controlling wheat damping-off disease

A solid culture was obtained by culturing *Gaeumannomyces graminis* (strain belonging to the genus *Gaeumannomyces*), which is a fungus causing wheat damping-off disease, on a barley medium, and the solid culture was mixed into commercially available gardening soil to a concentration of 0.5% (W/V). Further, each of the material produced in Example 1 (SD-F06 strain) and a Sterile Dark fungus (K89 strain) material produced in the same manner was mixed therein at a ratio shown in Table 2, and each of the mixtures was filled into a 1,000-ml plastic pot. Then, seeds of wheat (Norin 61) were sown in the plastic pot and cultivated in a greenhouse at 18°C to 24°C. After 30 day-cultivation, a pathogenesis degree was evaluated with five grades. As shown in Table 2, the preventive value in a 2.0% (W/V) Sterile Dark fungi-mixed area was 39.5%, on the other hand, the preventive value in a 1.0% (W/V) novel SD-F06 strain-mixed area was 57.9%, which revealed that the mixing of the SD-F06 strain even in a small amount exhibits a high preventive value.

### <Evaluation of disease incidence>

*Disease Incidence 0: no pathogenesis, 1: 10% pathogenesis, 2: 30% pathogenesis, 3: 50% pathogenesis, 4: 80% pathogenesis, and 5: 100% dead

[Table 2]

**Table 2 Controlling effect against wheat damping-off disease**

| | Disease Incidence (average) | Preventive value (%) |
|---|---|---|
| No treatment | 3.8 | - |
| Sterile Dark fungus (2.0% mixed area) Comparative Example 1 | 2.3 | 39.5 |
| SD-F06 strain (1.0% mixed area) Example 3 | 1.6 | 57.9 |

### Example 4

### Test of controlling Fusarium disease of lettuce

The SD-F06 strain material produced in Example 1 was mixed at 0.5% (W/V) into commercially available gardening soil, and then 800 ml of the mixture was filled into a 32-well cell tray (Naesaku-kun, manufactured by KOBAYASHI & CO., LTD.) and was irrigated. Seeds of lettuce (variety: Cisco) were sown therein and cultivated in a greenhouse at 18°C to 24°C. 14 days after sowing, a solid culture was obtained by culturing *Fusarium oxysporum* (fungus belonging to the genus *Fusarium*), which is a fungus causing lettuce root rot wilt disease, on a bran medium, and the solid culture was mixed into commercially available gardening soil to a concentration of 0.125% (W/V). The mixture was filled into a 1,000-ml plastic pot. Then, each of the seedlings cultivated in the cell tray was transplanted into the *Fusarium* fungi-contaminated pot. After 30 days, infected conditions of each lettuce were evaluated separately for an above-ground part and an underground part (root part). For the above-ground part, a disease incidence was evaluated by comparison with a healthy lettuce, with three grades by visual observation. For the underground part, after a main root thereof was divided into two parts with a razor, a browning degree of a duct was evaluated with three grades. As shown in Table 3, it was revealed that even the application of SD-F06 strain at concentration of 0.1% or 0.3% (W/V) exhibited sufficient controlling effect against a disease caused by a fungus belonging to the genus *Fusarium.* Further, it was also revealed that the controlling effect could still be maintained after transplant by only applying the SD-F06 strain at the time of producing seedlings.

### <Evaluation of disease incidence>

*Disease Incidence of above-ground part 0: no wilt, 1: slight wilt, 2: considerable wilt, 3: dead
*Disease incidence of underground part 0: no browning, 1: slight browning, 2: browning, 3: browning up to crown part and above
*Pathogenesis index = [Σ(number of plant with disease in each gradexdisease incidence)/number of investigated plant×3]×100

[Table 3]

**Table 3 Controlling effect against Fusarium disease of lettuce**

| SD-F06 strain application concentration (at the time of producing cell seedlings) | Above-ground part | | Underground part | |
|---|---|---|---|---|
| | Pathogenesis index | Preventive value (%) | Pathogenesis index | Preventive value (%) |
| 0% | 27 | - | 80 | - |
| 0.1% | 0 | 100 | 40 | 50.0 |
| 0.3% | 0 | 100 | 40 | 50.0 |
| 1% | 0 | 100 | 33 | 58.3 |

### Industrial Applicability

The novel filamentous fungus SD-F06 strain of the present invention can infect and live symbiotically with various plants at the inside of the root and has an ability to control a soil-borne disease, and thus is industrially useful. The material for controlling a soil-borne disease comprising a fungus body of the novel filamentous fungus SD-F06 strain of the present invention or a culture thereof is likewise industrially useful.

## Claims

1. A filamentous fungus SD-F06 strain (FERM BP-10841) or a mutant strain thereof, which has an ability of infecting a root of a plant and growing symbiotically with the plant and has an effect of controlling a soil-borne disease developed in the plant.

2. A material for controlling a soil-borne disease, comprising a fungus body or a fungal culture of the SD-F06 strain or the mutant strain thereof according to claim 1, as an active ingredient.

3. A method of controlling a soil-borne disease, comprising treating a soil for growing a plant with the material for controlling a soil-borne disease according to claim 2.

4. The material for controlling a soil-borne disease according to claim 2, wherein the soil-borne disease is caused by a phytopathogenic filamentous fungus.

5. The method of controlling a soil-borne disease according to claim 3, wherein the soil-borne disease is caused by a phytopathogenic filamentous fungus.

6. The material for controlling a soil-borne disease according to claim 4, wherein the disease caused by a phytopathogenic filamentous fungus is a disease caused by one or more kinds of fungi which belong to genera *Fusarium, Gaeumannomyces, Rhizoctonia, Pythium, Verticillium, Phytophthora, Sclerotium, Corticium, Plasmodiophora, Rhizopus, Trichoderma, Microdochium*, and *Sclerotinia.*

7. The method of controlling a soil-borne disease according to claim 5, wherein the disease caused by a phytopathogenic filamentous fungus is a disease caused by one or more kinds of fungi which belong to genera *Fusarium, Gaeumannomyces, Rhizoctonia, Pythium, Verticillium, Phytophthora, Sclerotium, Corticium, Plasmodiophora, Rhizopus, Trichoderma, Microdochium*, and *Sclerotinia*.


SEQUENCE LISTING

<110> IDEMITSU KOSAN CO., LTD.

<120> MATERIAL FOR CONTROL OF SOIL-BORNE DISEASE IN PLANT UTILIZING NOVEL FILAMENTOUS FUNGI

<130> 0600390-7185

<140> PCT/JP2007/067628
<141> 2007-09-11

<150> JP2006-251418
<151> 2006-09-15

<160> 1

<170> PatentIn version 3.3

<210> 1
<211> 1771
<212> DNA
<213> Ascomycota

<400> 1
gtagtcatat gcttgtctca aagattaagc catgcatgtc taagtataag caatctatac 60

ggtgaaactg cgaatggctc attaaatcag ttatcgttta tttgatagta ccttactact 120

tggataaccg tggtaattct agagctaata catgctaaaa acctcgactt cggaaggggt 180

gtatttatta gataaaaaac caatgccctt cggggctcct tggtgattca taataactta 240

acgaatcgca tggccttgtg ccggcgatgg ttcattcaaa tttctgccct atcaactttc 300

gatggtagga tagtggccta ccatggtttc aacgggtaac ggggaattag ggttctattc 360

cggagaggga gcctgagaaa cggctgccac atccaaggaa ggcagcaggc gcgcaaatta 420

cccaatcccg acacggggag gtagtgacaa taaatactga tacagggctc ttttgggtct 480

tgtaattgga atgagtacaa tttaaatccc ttaacgagga acaattggag ggcaagtctg 540

gtgccagcag ccgcggtaat tccagctcca atagcgtata ttaaagttgt tgcagttaaa 600

aagctcgtag ttgaaccttg aacctggctg gccggtccgc ctcaccgcgt gcactggtcc 660

ggccgggttt ttccttctgg ggagccgcat gcccttcact gggtgtgtcg gggaaccagg 720

acttttactt tgaaaaaatt agagtgttca aagcaggcct atgctcgaat acattagcat 780

ggaataatag aataggacgt gtggttctat tttgttggtt tctaggaccg ccgtaatgat 840

taatagggat agtcgggggc atcagtattc aattgtcaga ggtgaaattc ttggatttat 900

tgaagactaa ctactgcgaa agcatttgcc aaggatgttt tcattaatca gtgaacgaaa 960

gttaggggat cgaagacgat cagataccgt cgtagtctta accataaact atgccgacta 1020

gggatcgggc gatgttattt ttttgactcg ctcggcacct tacgagaaat caaagtcttt 1080

gggttctggg gggagtatgg tcgcaaggct gaaacttaaa gaaattgacg gaagggcacc 1140

accaggagtg gagcctgcgg cttaatttga ctcaacacgg ggaaactcac caggtccaga 1200

cacaataagg attgacagat tgagagctct ttcttgattt tgtgggtggt ggtgcatggc 1260

cgttcttagt tggtggagtg atttgtctgc ttaattgcga taacgaacga gaccttaacc 1320

tgctaaatag ccaggctagc tttggctggt cgccggcttc ttagagggac tatcggctca 1380

agccgatgga agtttgaggc aataacaggt ctgtgatgcc cttagatgtt ctgggccgca 1440

cgcgcgctac actgacagag ccaacgagtt tcttccttgt ccgaaaggtc tgggtaatct 1500

tgttaaactc tgtcgtgctg gggatagagc attgcaatta ttgctcttca acgaggaatt 1560

cctagtaagc gcaagtcatc agcttgcgct gattacgtcc ctgccctttg tacacaccgc 1620

ccgtcgctac taccgattga atggctaagt gaggcctttg gaccggccca gagagggtgg 1680

caacacccac tcagggctgg aaaatcgtac aaacttggtc atttagagga agtaaaagtc 1740

gtaacaaggt ttccgtaggt gaacctgcgg a 1771